# EUROPEAN PATENT APPLICATION

(11) **EP 4 389 135 A1**
(43) Date of publication of application: **26.06.2024**
(21) Application number: 22858629.3
(22) Date of filing: 29.07.2022
(51) Int. Cl.: A61K 36/25, A61P 27/02, A23L 33/105

(54) **EYE DAMAGE PREVENTION COMPOSITION CONTAINING DENDROPANAX MORBIFERA LEV. EXTRACT**

(30) Priority: 20.08.2021 KR 20210110026
(71) Applicant: Korea Ginseng Corp., Daejeon 34337 (KR)
(72) Inventor: IN, Gyo, Daejeon 34128 (KR); SO, Seung-Ho, Daejeon 34128 (KR); KIM, Jong Han, Daejeon 34128 (KR); JANG, Kyoung Hwa, Daejeon 34128 (KR); KOO, Gi-Bang, Daejeon 34128 (KR); KWON, Han Ol, Daejeon 34128 (KR); JEONG, Yoonseon, Daejeon 34128 (KR); HAN, Byung Cheol, Daejeon 34128 (KR)
(74) Representative: Maiwald GmbH
(86) International application number: PCT/KR2022/011175
(87) International publication number: WO 2023/022390

(57) **Abstract**

A composition containing a *Dendropanax morbifera* LEV. extract, of the present invention, has excellent activities of inhibiting the oxidation of A2E, inhibiting oxidized A2E-induced death of retinal pigmented epithelial cells, and inhibiting the accumulation of drusen, and thus can be used for preventing eye damage. In addition, a composition containing a *Dendropanax morbifera* LEV. extract, of the present invention, can be developed even into a therapeutic agent for various eye diseases and a health functional food.

## Description

### TECHNICAL FIELD

The present invention relates to a composition containing a *Dendropanax morbifera* extract as an active ingredient and use thereof.

### BACKGROUND ART

Since the eye is the most important sensory organ, functional impairment or loss of the eye is one of the biggest factors that reduce the quality of life. Modern people spend most of their time in front of various digital screens such as computers and smart phones, and as LEDs are frequently used on smart device screens, blue light is deteriorating eye health.

Blue light is a light taking on a blue color in the range of 400 nm to 500 nm among the visible light, and when exposed to blue light for a long time, it stimulates the optic nerve, causing eye fatigue and becomes the cause of various eye diseases. Since blue light has high energy and high penetrating power, upon entering the eye as-is, it makes the focus on the retina opaque and reduces sharpness, and chronic exposure to blue light causes aging and degeneration of the retina. It is known that the influences of blue light on the human body include dry eye and eye fatigue, a decrease in visual acuity and various eye diseases, accelerated retinal aging, macular degeneration, and insomnia due to suppression of melatonin production (Ganka Gakkai Zasshi. 2001 Oct; 105 (10) :687-95; Archives of Ophthalmology 1992; 110:99-104; Review of Ophthalmology Oct 15 2003; 10(10)).

It is known that metabolic waste by-products accumulate in retinal cells throughout life due to the action of blue light, and in particular, the accumulation occurs most actively in retinal pigment epithelial cells under the center of the retina where rod cell photoreceptors are highly concentrated. One of the most important things among these metabolic waste by-products is *N*-retinylidene-*N*-retinyl-ethanolamine (A2E), a component of lipofuscin, and a high A2E concentration becomes the main cause involved in the death of retinal pigment epithelial cells. In addition, A2E is photooxidized by blue light even at a low concentration and is changed into peroxy-A2E and furano-A2E in the form of epoxide. The highly reactive epoxide form of A2E causes oxidative stress and inflammatory responses and alters mitochondrial protein activity, thereby inducing apoptosis. As a result, loss of the function of normal retinal pigment epithelial cells leads to secondary death of photoreceptor cells. In particular, in the case of the macula, in which photoreceptor cells are densely concentrated, it is seriously damaged, resulting in macular degeneration. That is, macular degeneration differs from diabetic retinopathy in terms of the cause of disease in that macular degeneration is caused by the oxidation of A2E due to cellular oxidative stress in the retina caused by blue light, whereas diabetic retinopathy is caused by the action of glycated products due to high blood sugar levels and angiogenic factors such as VEGF.

Macular degeneration may be divided into two types: dry (non-exudative) macular degeneration and wet (exudative) macular degeneration. Dry macular degeneration accounts for about 90% of all patients with macular degeneration, and dry macular degeneration is characterized in that a waste material called drusen is accumulated between the retinal pigment epithelium and Bruch's membrane, resulting in atrophy or thinning of the macular tissue. In contrast, wet macular degeneration accounts for about 10% of all patients with macular degeneration, and it refers to a condition in which normal blood and nutrients are not supplied to the macula, resulting in formation of abnormal new blood vessels and rupture of the same, and thus causes blood or mucus to leak into the macula, thereby causing serious vision loss. Since early symptoms of dry macular degeneration are asymptomatic or even if symptoms are present, it is easy to neglect because it is considered presbyopia. However, if the progression of dry macular degeneration is not inhibited or treated, vision loss may occur due to continuous retinal damage, and it may progress to wet macular degeneration and lead to blindness; therefore, it is important that dry macular degeneration be prevented in the early stage.

Previously released eye health-related products are synthetic treatments, which cause side effects due to long-term use of synthetic compounds. Accordingly, there is a need for the development of eye health-improving materials derived from natural products that are conventionally consumed as food instead of artificially synthesized specific compounds.

### DISCLOSURE OF THE INVENTION

### TECHNICAL PROBLEM

An object of the present invention is to provide a composition for preventing eye damage caused by blue light.

Another object of the present invention is to provide a health functional food for preventing or improving eye damage caused by blue light.

Still another object of the present invention is to provide a composition for preventing or treating eye diseases caused by blue light.

### TECHNICAL SOLUTION

In order to achieve the above objects, an aspect of the present invention provides a composition for preventing eye damage containing a *Dendropanax morbifera* extract as an active ingredient.

Another aspect of the present invention provides a health functional food for preventing or improving eye damage containing a *Dendropanax morbifera* extract as an active ingredient.

Still another aspect of the present invention provides a pharmaceutical composition for preventing or treating eye diseases containing a *Dendropanax morbifera* extract as an active ingredient.

### ADVANTAGEOUS EFFECTS

The composition containing a *Dendropanax morbifera* extract of the present invention, has excellent activities of inhibiting the oxidation of A2E caused by blue light, inhibiting oxidized A2E-induced death of retinal pigment epithelial cells, and inhibiting the accumulation of drusen, and thus can be used for preventing eye damage caused by blue light. In addition, the composition containing a *Dendropanax morbifera* extract of the present invention can be developed even into a therapeutic agent for various eye diseases caused by blue light and a health functional food.

The effects of the present invention are not limited to the effects mentioned above, and other effects not mentioned will be clearly understood by those skilled in the art from the following description.

### BRIEF DESCRIPTION OF THE DRAWINGS

FIG. 1 is a graph showing that photo-oxidation of A2E, which was increased by blue light treatment in human retinal pigment epithelial cells ARPE-19, was significantly inhibited by treating with a *Dendropanax morbifera* extract.
FIG. 2 is a graph showing the effect of inhibiting drusen accumulation by treatment with a *Dendropanax morbifera* extract in human retinal pigment epithelial cells ARPE-19.
FIG. 3 is a graph showing that the cell viability reduced by blue light treatment in human retinal pigment epithelial cells ARPE-19 was significantly increased by treating with a *Dendropanax morbifera* extract.
FIG. 4 shows images of the thickness of the outer nuclear layer (ONL) of the retina after administering a test drug to an animal model, in which loss of optic nerve cells similar to dry macular degeneration was induced, observed by a microscope.
FIG. 5 is a graph showing that the thickness of the outer layer of the retina, which was reduced by excessive irradiation of a light source in an animal model where loss of optic nerve cells similar to dry macular degeneration was induced, was increased by treating with a *Dendropanax morbifera* extract.
FIG. 6 is graphs showing that the activity of visual cells, which was reduced by excessive irradiation of a light source in an animal model where loss of optic nerve cells similar to dry macular degeneration was induced, was increased by treating with a *Dendropanax morbifera* extract.
FIG. 7 shows images of the thickness of the outer nuclear layer (ONL) of the retina after administering a test drug to an animal model, in which loss of optic nerve cells similar to dry macular degeneration was induced by administering N-methyl-N-nitrosourea (MNU), observed by a microscope.
FIG. 8 is a graph showing that the thickness of the outer nuclear layer of the retina, which was reduced by the administration of N-methyl-N-nitrosourea in an animal model where loss of optic nerve cells similar to dry macular degeneration was induced, was increased by treating with a *Dendropanax morbifera* extract.
FIG. 9 shows images of the position, in which the folding deformation of retinal tissue occurred by administering a test drug to an animal model where morphological deformation was induced in the retinal tissue positioned in an upper part thereof by administering sodium iodate (NaIO₃), observed by a microscope.
FIG. 10 is a graph showing that the damage of retinal pigment epithelial cells, which was increased by the administration of sodium iodate (NaIO₃) in an animal model where morphological deformation was induced in the retinal tissue positioned in an upper part thereof by the administration of sodium iodate, was reduced by treating with a *Dendropanax morbifera* extract.
FIG. 11 shows images of the cells, in which a TUNEL-positive reaction occurred in the cell nuclear layer of the optic nerve by administering a test drug to an animal model where loss of optic nerve cells similar to dry macular degeneration was induced, observed by a microscope.
FIG. 12 is a graph showing that the increased apoptotic response in an animal model, where loss of optic nerve cells similar to dry macular degeneration was induced, was reduced by treating with a *Dendropanax morbifera* extract.
FIG. 13 is a graph showing that the expression of Bax, which is a pro-apoptotic factor that induces apoptosis, was decreased, the expression of Bcl-2, which is the anti-apoptotic factor, was increased, and the increased expression of the cleaved form of caspase-3 was decreased by treating with a *Dendropanax morbifera* extract in an animal model where loss of optic nerve cells similar to dry macular degeneration was induced.
FIG. 14 shows images of ZO-1, which is a protein constituting a tight junction, after administering a test drug to an animal model where morphological deformation was induced in the retinal tissue positioned in an upper part thereof, observed by a microscope.
FIG. 15 a diagram showing the mechanism of action of a *Dendropanax morbifera* extract to improve macular degeneration.

### MODE FOR CARRYING OUT THE INVENTION

Hereinafter, the present invention will be described in detail.

An aspect of the present invention provides a composition for preventing eye damage containing a *Dendropanax morbifera* extract as an active ingredient.

The *"Dendropanax morbifera"* belongs to the Family *Araliaceae,* and certainly includes subspecies thereof and variants thereof. In addition, *Dendropanax morbifera* of the present invention includes herbal medicines of the same genus that are obvious in the art and may be used for the same or similar purposes of prevention, improvement, and treatment purposes of the present invention.

The "extract" includes the extracts themselves, and extracts of all formulations that may be formed using a liquid extract, such as an extract obtained by the extraction treatment of *Dendropanax morbifera,* a diluted or concentrated solution of the extract, a dried product obtained by drying the extract, a crude or purified product of the extract, and a mixture thereof. For example, the extract of the present invention may be prepared in the form of dry powder after extraction and used.

The *Dendropanax morbifera* extract may be extracted from natural, hybrid, or variant plants of a *Dendropanax morbifera* tree, and may also be extracted from plant tissue culture. In addition, the *Dendropanax morbifera* extract may be extracted from the leaves, stems, roots, flowers, and seeds of a *Dendropanax morbifera* tree.

The method for extracting a *Dendropanax morbifera* extract is not particularly limited and may be extracted according to a method commonly used in the art. Non-limiting examples of the extraction method may include a hot water extraction method, an ultrasonic extraction method, a filtration method, a reflux extraction method, etc., which may be performed alone or in combination of two or more methods.

The type of extraction solvent used to extract the *Dendropanax morbifera* extract is not particularly limited, and any solvent known in the art may be used. Non-limiting examples of the extraction solvent may include C₁₋₄ lower alcohols such as water, methanol, ethanol, propyl alcohol, and butyl alcohol; polyhydric alcohols such as glycerin, butylene glycol, and propylene glycol; hydrocarbon-based solvents such as methyl acetate, ethyl acetate, acetone, benzene, hexane, diethyl ether, and dichloromethane; or mixtures thereof. For example, the *Dendropanax morbifera* extract of the present invention may be extracted from the leaves and stems of *Dendropanax morbifera* with water, C₁₋₄ lower alcohol or a mixed solvent thereof.

The "eye damage" may be caused by blue light, and may specifically mean eye damage caused by photo-oxidation of A2E by blue light and accumulation thereof. More specifically, the eye damage may mean eye damage caused by oxidative damage of the eye, aging of the eye, eye fatigue, deterioration of an eye function, or an eye disease, but is not limited thereto.

The eye disease may be an eye disease caused by blue light, and may specifically mean a retinal disease associated with photooxidation of A2E by blue light and accumulation of drusen. Specifically, the eye disease may mean one or more types selected from the group consisting of retinal degeneration, macular degeneration, glaucoma, retinitis pigmentosa, Stargardt disease, choroideremia, gyrate-atrophy, retinal detachment, optic neuropathy, and dry eye syndrome, but is not limited thereto.

In a specific embodiment of the present invention, in order to confirm the effect of a *Dendropanax morbifera* extract on preventing eye damage caused by blue light, retinal pigment epithelial cells were treated with a *Dendropanax morbifera* extract together with blue light to measure the degree of photooxidation of A2E, and as a result, it was confirmed that the increased photooxidation of A2E by blue light was apparently inhibited by the treatment with the *Dendropanax morbifera* extract.

In addition, in order to confirm the effect of preventing cell damage caused by oxidized A2E, retinal pigment epithelial cells were treated with a *Dendropanax morbifera* extract along with blue light to confirm cell viability, and as a result, it was confirmed that the decreased cell viability by blue light was apparently increased by the treatment with the *Dendropanax morbifera* extract.

In addition, as a result of confirming the degree of drusen accumulation by irradiating blue light, it was confirmed that drusen accumulation was reduced by the treatment of a *Dendropanax morbifera* extract.

In addition, it was confirmed that the treatment with a *Dendropanax morbifera* extract increased the thickness of the outer nuclear layer of the retina, which was reduced by light irradiation, and improved the decrease in activity of visual cells.

In addition, it was confirmed that damage or deformation of retinal pigment epithelial cells was improved by inhibiting deformation of retinal tissue by treating with a *Dendropanax morbifera* extract in an animal model where macular degeneration was induced by a drug.

Further, it was confirmed that treatment with a *Dendropanax morbifera* extract inhibits retinal cell death induced by light irradiation to thereby improve damage of optic nerve cells and maintains tight junctions of retinal pigment epithelial cells to thereby improve damage of retinal pigment epithelial cells.

Therefore, it is apparent that the *Dendropanax morbifera* extract prevents damage to retinal pigment epithelial cells by inhibiting the oxidation of A2E, and has the activity of inhibiting the atrophy or morphological change in retinal tissue by inhibiting the accumulation of drusen, and thus, the *Dendropanax morbifera* extract may be effectively used as an active ingredient in a composition for preventing eye damage.

Another aspect of the present invention provides a health functional food for preventing or improving eye damage containing a *Dendropanax morbifera* extract as an active ingredient.

The *Dendropanax morbifera,* extract, and eye damage are as described above.

Since *Dendropanax morbifera* has been used as a natural material for a long time and has been proven to be safe for the human body, it may be prepared and consumed in the form of food that may be able to prevent or improve eye damage while being normally consumed.

The "prevention" means all actions which inhibit or delay eye damage by ingestion of the health functional food.

The "improvement" means all actions which reduce the parameters related to the condition to be treated by ingestion of the health functional food, for example, the severity of symptoms.

The health functional food may include sitologically acceptable food supplement additives in addition to a *Dendropanax morbifera* extract.

The food supplement additive means a component that may be supplementally added to food, and may be appropriately selected and used by those skilled in the art as one being added to prepare a health functional food of each formulation. Examples of supplemental food additives include various nutrients, vitamins, minerals (electrolytes), flavors (e.g., synthetic flavors and natural flavors), colorants and fillers, pectic acid and salts thereof, alginic acid and salts thereof, organic acids, protective colloidal thickeners, pH adjusters, stabilizers, preservatives, glycerin, alcohol, carbonating agents used in carbonated beverages, etc., but are not limited thereto.

The health functional food means food prepared and processed in the form of tablets, capsules, powders, granules, liquids, pills, etc. using raw materials or components having useful functionalities for the human body. In particular, functionality means obtaining useful effects for health purposes with respect to the structure and functions of the human body, such as adjusting nutrients or physiological functions. The health functional food of the present invention may be prepared by a method commonly used in the art, and may be prepared by adding raw materials and components commonly added in the art during the preparation. In addition, the formulation of the health functional food may also be prepared without limitation as long as the formulation is recognized as a health functional food.

The health functional food may be prepared in various types of formulations, and unlike general drugs, it has the advantage in that it has no side effects that may occur when taking drugs for a long time because of using food as a raw material, and has excellent portability; therefore, the health of the present invention functional food may be taken as a supplement to enhance the effect of improving eye damage.

There is no limitation with regard to the form that the health functional food may take, and it may include all foods in a conventional sense and may be used interchangeably with terms known in the art, such as functional food. In addition, the health functional food of the present invention may be prepared by mixing other suitable auxiliary ingredients and known additives that may be included in food according to the selection of those skilled in the art. Examples of the food that may be added include meats, sausage, bread, chocolate, candies, snacks, confectioneries, pizza, ramen, other noodles, chewing gums, dairy products including ice cream, various soups, beverages, tea, drinks, alcoholic beverages, vitamin complexes, etc., and the food may be prepared by adding the extract according to the present invention, as a main ingredient, to juice, tea, jelly, juice, etc. It also includes food used as feed for animals.

Still another aspect of the present invention provides a pharmaceutical composition for preventing or treating eye diseases containing a *Dendropanax morbifera* extract as an active ingredient.

The *Dendropanax morbifera* extract is as described above.

The "prevention" means all actions which inhibit or delay the occurrence of eye diseases by administering the composition according to the present invention.

The "treatment" means all actions which improve or beneficially change the symptoms of eye diseases by administering the composition according to the present invention.

The "eye disease" may be an eye disease caused by blue light, and may specifically mean a retinal disease associated with photooxidation of A2E by blue light and accumulation thereof, or accumulation of drusen and photooxidation. Specifically, the eye disease may mean one or more types selected from the group consisting of retinal degeneration, macular degeneration, glaucoma, retinitis pigmentosa, Stargardt disease, choroideremia, gyrate-atrophy, retinal detachment, optic neuropathy, and dry eye syndrome, but is not limited thereto.

The macular degeneration (MD) means a phenomenon in which central vision is destroyed by damage to the macula, and the macular degeneration diseases may include dry macular degeneration, wet macular degeneration, age-related macular degeneration, myopic macular degeneration, idiopathic macular degeneration, etc. and specifically, it may be age-related macular degeneration (AMD).

Although the exact etiology of age-related macular degeneration has not yet been certainly identified, it is generally known that accumulation of excessive pigment materials in retinal pigment epithelial cells due to aging appears in the early stage of age-related macular degeneration, and A2E, which is produced by the synthesis of all-trans-retinal and ethanolamine, is a material that typically accumulates in retinal epithelial cells, and it generates singlet oxygen by blue light and may cause damage to retinal pigment epithelial cells while oxidizing carbon-to-carbon double bonds.

Prevention or treatment of the eye disease may be achieved by inhibiting apoptosis caused by A2E oxidized by blue light or inhibiting accumulation of drusen. In a specific embodiment of the present invention, it was confirmed that apoptosis occurred when blue light was irradiated after A2E accumulation in retinal pigment epithelial cells, but treatment with a *Dendropanax morbifera* extract has the effect of inhibiting apoptosis. In addition, it was confirmed that when retinal pigment epithelial cells were treated with a *Dendropanax morbifera* extract, the accumulation of drusen was inhibited. That is, the *Dendropanax morbifera* extract of the present invention may be usefully used for preventing or treating eye diseases by inhibiting the death of retinal pigment epithelial cells caused by exposure to blue light or inhibiting the accumulation of drusen.

The pharmaceutical composition includes a therapeutically effective amount of a *Dendropanax morbifera* extract; and a pharmaceutically acceptable carrier.

The "therapeutically effective amount" means an amount sufficient for the composition of the present invention to achieve treatment or prevention of eye diseases caused by blue light.

The pharmaceutically acceptable carrier, which is one commonly used in preparing formulations, may include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, etc., but is not limited thereto.

The pharmaceutical composition may be administered orally or parenterally, and in the case of parenteral administration, intravenous injection, subcutaneous injection, intramuscular injection, intraperitoneal injection, or transdermal administration may be used.

A suitable dosage of the pharmaceutical composition varies depending on factors such as a method of formulation, a method of administration, patient's age, body weight, sex, pathological condition, food, administration time, administration route, excretion rate, and response sensitivity, and usually skilled practitioners may be able to easily determine and prescribe doses effective for the desired treatment or prevention.

The daily dose of the pharmaceutical composition is 0.0001 mg/kg to 100 mg/kg, and preferably 0.001 mg/kg to 30 mg/kg, which may be administered once or several times a day. In addition, the administration period may be 1 day to 2 months, but it may be administered without limitation until the effect of preventing or treating a disease appears.

The pharmaceutical composition is prepared in unit dose form by formulating using a pharmaceutically acceptable carrier and/or excipient according to a method that may easily be performed by those skilled in the art, or it may be prepared by placing the same in a multi-dose container. In particular, the formulation may be in the form of a solution, suspension, or emulsion in an oil or aqueous medium, or may be in the form of an extract, powder, granule, tablet, or capsule, and may further contain a dispersing agent or stabilizer.

The pharmaceutical composition may also be formulated as ophthalmic compositions such as eye drops and eye ointments. This form may include all ophthalmic preparations for topical administration to the eye used in the field of ophthalmology. Eye drops are prepared by dissolving active ingredients in a sterile aqueous solution such as saline and buffer. Eye drops may be provided as a powder composition to be dissolved prior to use, or may be provided by combining with a powder composition to be dissolved prior to use. Eye ointments may be prepared by mixing active ingredients into an ointment base.

Hereinafter, the present invention will be described in detail by Examples and Experimental Examples. However, the following Examples and Experimental Examples are only for illustrating the present invention, and the content of the present invention is not limited by the following Examples and Experimental Examples.

### Embodiments for Carrying Out the Invention

### [Preparation Example]

### Preparation of Dendropanax morbifera extract

To 2 kg of leaves and branches of a *Dendropanax morbifera* tree, 15 times the weight of 300 (v/v) ethanol aqueous solution was added, extraction was performed 1-3 times at 80 °C for 8 hours, and the extracts obtained from each extraction were mixed. The extract was filtered using a 5 µm filter. The filtrate was concentrated at 55 °C using a rotary vacuum concentrator (BUCHI, R-220). The concentrate was diluted to brix 20 by adding sterilized distilled water thereto, and lyophilized to obtain 210 g of a *Dendropanax morbifera* extract powder.

### [Experimental Example 1]

### Photooxidation inhibitory effect of A2E by a Dendropanax morbifera extract

A2E accumulated in retinal pigment epithelial cells, which is a causative agent of macular degeneration, is known to be deposited in retinal pigment epithelial cells, in which photooxidation occurs when irradiated with blue light to thereby induce cytotoxicity. In order to confirm whether a *Dendropanax morbifera* extract has the effect of inhibiting photooxidation of A2E, ARPE-19 cells, which are human retinal pigment epithelial cell lines, and A2E were used.

Specifically, ARPE-19 cells (CRL-2302, ATCC, USA) were treated with 90 µM A2E (Gene and cell technologies, USA), and then treated with a *Dendropanax morbifera* extract prepared in Preparation Example above at 0 µg/mL, 7.8 µg/mL, 15.6 µg/mL, 31.25 ug/mL, 62.5 µg/mL, 125 µg/mL, and 250 µg/mL, and then irradiated with blue light (430 nm, intensity: 2.0 mW/cm² to 2.5 mW/cm²) for 10 minutes. In order to measure the degree that A2E is photooxidated, the absorbance at 440 nm was measured using a micro plate leader before and after the irradiation with blue light.

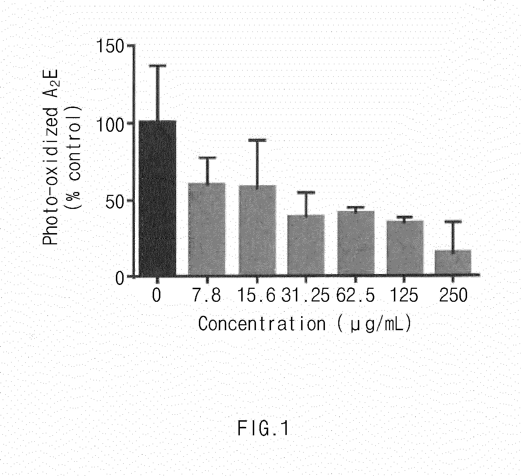

As a result, as shown in FIG. 1, in the group treated with a *Dendropanax morbifera* extract, the accumulation of oxidized A2E was inhibited by 40% to 75% compared to the negative control group not treated with a *Dendropanax morbifera* extract.

From the above, it can be seen that the *Dendropanax morbifera* extract has the effect of inhibiting the accumulation of A2E in cells and inhibiting photo-oxidation of A2E.

### [Experimental Example 2]

### Effects of inhibiting photooxidation and accumulation of drusen by a Dendropanax morbifera extract

It is known that when drusen accumulates in retinal epithelial cells, macular tissue becomes thin or atrophied, resulting in damage to a visual function and visual acuity, thereby inducing macular degeneration. After photooxidation, drusen produces active dicarbonyl species such as glyoxal and methylglyoxal (MGO), which are cytotoxic materials, in retinal cells by peroxy-A2E, which then bind non-specifically to intracellular proteins to form MGO-modified adducts that are accumulated in retinal cells. Since these MGO-modified adducts are also known to aggravate retinopathy by acting as a component of drusen, it was checked whether a *Dendropanax morbifera* extract inhibits photooxidation of drusen and accumulation of MGO-modified adducts.

Specifically, 0.7 mL of bovine serum albumin at 10 mg/mL (Sigma, St. Louis, MO, USA) dissolved in 50 mM phosphate buffer (pH 7.4) and 0.1 mL of 0.22 M methylglyoxal were added and treated with the *Dendropanax morbifera* extract prepared in Preparation Example at 0 µg/mL, 7.8 µg/mL, 15.6 µg/mL, 31.25 µg/mL, 62.5 µg/mL, 125 µg/mL, and 250 µg/mL to a total volume of 1 mL. The resultants were left at 37 °C for 7 days to induce the production of a glycation product. After one week, the absorbance at 450 nm was measured using a micro plate leader.

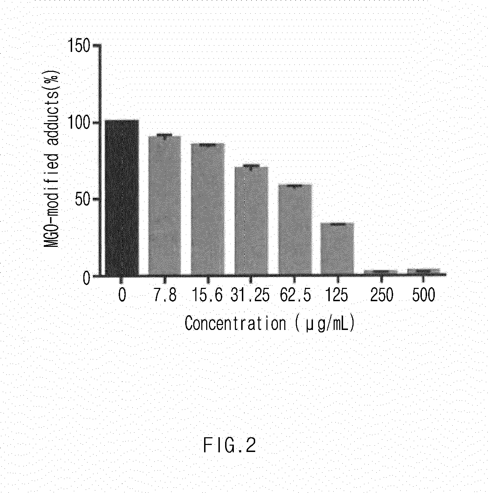

As a result, as shown in FIG. 2, in the group treated with a *Dendropanax morbifera* extract, the accumulation of MGO-modified adducts was inhibited compared to the negative control group not treated with a *Dendropanax morbifera* extract.

From the above, it can be seen that the *Dendropanax morbifera* extract has the effects of preventing eye damage by inhibiting photooxidation of drusen and accumulation of MGO-modified adducts, and inhibiting the occurrence and deterioration of macular degeneration.

### [Experimental Example 3]

### Effect of Dendropanax morbifera extract on protection of retinal pigment epithelial cells against blue light-induced photooxidation

Considering that the A2E accumulated in retinal pigment epithelial cells causes photooxidation when irradiated with blue light, which induces cytotoxicity that leads to apoptosis, it was confirmed whether a *Dendropanax morbifera* extract can inhibit cytotoxicity and apoptosis induced by blue light.

Specifically, ARPE-19 cells were treated with the *Dendropanax morbifera* extract prepared in Preparation Example above at 0 µg/mL and 50 µg/mL, chlorogenic acid at 50 µg/mL, neochlorogenic acid at 50 µg/mL, stigmasterol at 50 µg/mL or lutein at 50 µg/mL for 24 hours, then treated with 20 µM of A2E for 24 hours, and then irradiated with blue light for 10 minutes. Thereafter, the cell viability of ARPE-19 cells was measured with a cell counting kit (Dojindo Labs, Japan), and the cell viability of each treatment group relative to the control group, in which A2E was accumulated and blue light was not irradiated, was expressed in percentage (%).

As a result, the cell viability of the negative control group, in which A2E was accumulated and which was irradiated with blue light, was significantly reduced compared to the control group, and apoptosis was induced. In contrast, it was confirmed that the cell viability in the group treated with a *Dendropanax morbifera* extract was increased compared to the negative control group treated with only A2E, and it was shown that the cell viability was increased even compared to the positive control group treated with lutein.

From the above, it can be seen that the *Dendropanax morbifera* extract prevents or treats macular degeneration by inhibiting apoptosis caused by accumulation and oxidized A2E in retinal pigment epithelial cells.

### [Experimental Example 4]

### Effect of Dendropanax morbifera extract on protection of retinal pigment epithelial cells against blue light-induced photooxidation

Considering that the A2E accumulated in retinal pigment epithelial cells causes photooxidation when irradiated with blue light, which induces cytotoxicity that leads to apoptosis, it was confirmed whether a *Dendropanax morbifera* extract can inhibit cytotoxicity and apoptosis induced by blue light.

Specifically, ARPE-19 cells were treated with the *Dendropanax morbifera* extract prepared in Preparation Example above at 0 µg/mL and 50 µg/mL, chlorogenic acid at 50 µg/mL, neochlorogenic acid at 50 µg/mL, stigmasterol at 50 µg/mL or lutein at 50 µg/mL for 24 hours, then treated with 20 µM of A2E for 24 hours, and then irradiated with blue light for 10 minutes. Thereafter, the cell viability of ARPE-19 cells was measured with a cell counting kit (Dojindo Labs, Japan), and the cell viability of each treatment group relative to the control group, in which A2E was accumulated and blue light was not irradiated, was expressed in percentage (%).

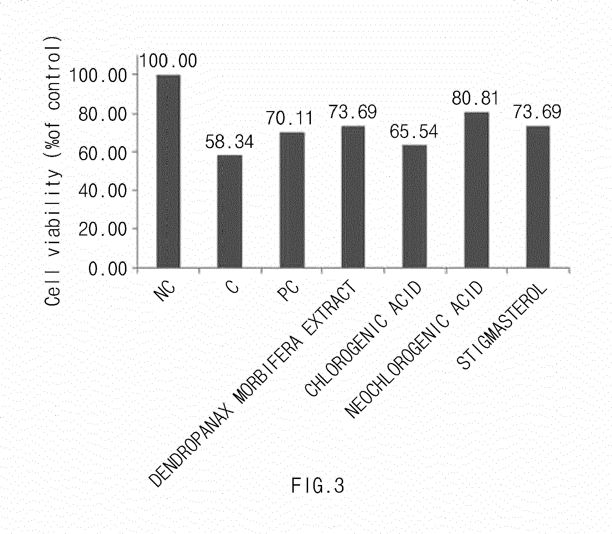

As a result, as shown in FIG. 3, the cell viability of the negative control group, in which A2E was accumulated and which was irradiated with blue light, was significantly reduced compared to the control group, and apoptosis was induced. In contrast, it was confirmed that the cell viability in the group treated with a *Dendropanax morbifera* extract was increased compared to the negative control group treated with only A2E, and it was shown that the cell viability was increased even compared to the positive control group treated with lutein.

From the above, it can be seen that the *Dendropanax morbifera* extract prevents or treats macular degeneration by inhibiting apoptosis caused by accumulation and oxidized A2E in retinal pigment epithelial cells.

### [Experimental Example 5]

### Effect of Dendropanax morbifera extract on improving damage or deformation of photoreceptor cells in macular degeneration animal model

When drusen accumulates in the macular area due to dry macular degeneration, the macular area is photooxidized and causes damage to surrounding tissues and optic nerve cells, thereby resulting in a decrease of visual acuity, and in severe cases, geographic atrophy occurs, a condition in which optic nerve cells are lost and retinal apoptosis is induced. In order to determine whether a *Dendropanax morbifera* extract has the effect of improving damage or deformation of photoreceptor cells in an animal model where optic nerve cell damage was caused by excessive irradiation of a light source including blue light and loss of optic nerve cells similar to dry macular degeneration, the changes in the thickness of outer nuclear layer (ONL) of the retina were measured.

Specifically, 6-week-old male BALB/c mice (Orient Bio Inc.) were acclimatized for one week and then subjected to dark adaptation in a dark place without light for 14 hours from the evening before white LED exposure containing blue light, and then, their pupils were dilated with 1% tropicamide eye drop the next morning. Thereafter, the mice were exposed for 0.5 hours to a specially manufactured cooled white LED light panel with 5,000 lux brightness and then placed in a dark room for 24 hours. After exposure to light, the mice were orally administered with a test drug once daily for 7 days. The concentrations of the drugs administered are shown in Table 1 below. Neochlorogenic acid, which is known to help prevent macular degeneration by activating aged retinal nerve cells, and lutein, which is widely known as a component of eye nutrients, were set as positive controls, and the *Dendropanax morbifera* extract according to Preparation Example above was administered at various concentrations. After completion of drug administration, autopsies were performed to extract the eyeballs, which were fixed in 10% neutralized formalin for one day, and then embedded in paraffin to prepare slide sections. The slide sections were stained with hematoxylin & eosin (H&E) and observed under an optical microscope, and damage or deformation of photoreceptor cells was evaluated by measuring changes in the thickness of the outer nuclear layer of retinal tissue.

**[Table 1]**

| **Test Group** | **Group Name** | **Drug Administered** | **Dose Concentration (mg/kg/day)** | **No. of Mice** |
|---|---|---|---|---|
| Normal Control Group | Normal | - | - | 8 |
| Negative Control Group | LRD | - | - | 8 |
| Group Administered with *Dendropanax morbifera* Extract | DM-25 | *Dendropanax morbifera* extract | 25 | 8 |
| Group Administered with *Dendropanax morbifera* Extract | DM-50 | *Dendropanax morbifera* extract | 50 | 8 |
| Group Administered with *Dendropanax morbifera* Extract | DM-100 | *Dendropanax morbifera* extract | 100 | 8 |
| Group Administered with *Dendropanax morbifera* Extract | DM-200 | *Dendropanax morbifera* extract | 200 | 8 |
| Positive Control Group | NCA-6.5 | Neochlorogenic acid | 6.5 | 8 |
| Positive Control Group | Lutein-20 | Lutein | 20 | 8 |

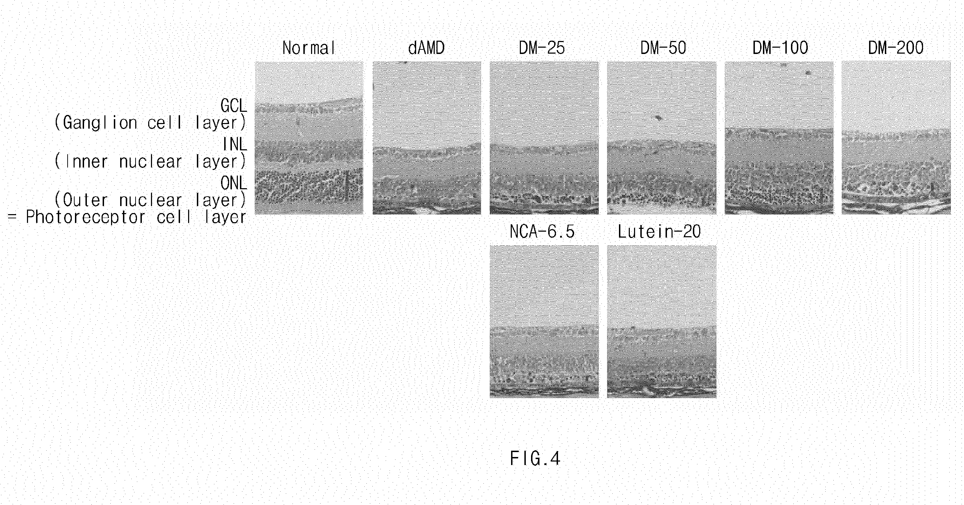

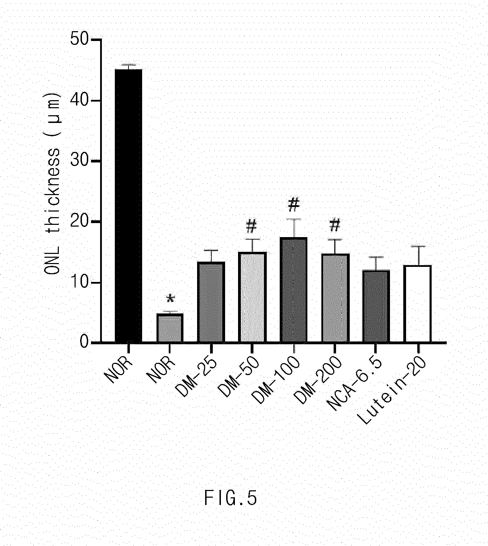

As a result, as shown in FIGS. 4 and 5, in the negative control group, the thickness was decreased while the cell nuclei of the outer nuclear layer (ONL) containing the nuclei of optic nerve cells was decreased, and when a *Dendropanax morbifera* extract was administered, it was shown that the thickness of the outer nuclear layer was increased in a concentration-dependent manner, and its effect was superior to that of lutein. However, the effect of increasing the thickness of the outer nuclear layer was slightly decreased in the experimental group administered with 200 mg/kg of the *Dendropanax morbifera* extract compared to the experimental group administered with 100 mg/kg of the *Dendropanax morbifera* extract, which is presumed to be a phenomenon in which the absorption of effective materials is competitively inhibited by various phytocompounds contained in the extract as the concentration of the *Dendropanax morbifera* extract increased. The thickness of the outer nuclear layer was also increased in the group administered with neochlorogenic acid, which was another positive control group, but the result was not statistically significant.

From the above, it can be seen that the *Dendropanax morbifera* extract has the effect of improving the damage or deformation of photoreceptor cells by increasing the thickness of the outer nuclear layer (ONL) of the retina.

### [Experimental Example 6]

### Confirmation of effect of Dendropanax morbifera extract on improving the decrease of visual cell activity in animal model with macular degeneration

An electroretinogram (ERG) is a degree of electric potential of the retinal optic nerve representing an electrical signal generated by a response of the optic nerve to light, and is used as an index to determine the presence/absence of retinal damage or disease. The electroretinogram consists of wave a and wave b, in which the wave a is generated by photoreceptors, and the wave b is generated by depolarization of bipolar cells in the inner nuclear layer of the retina by light stimulation. In order to confirm whether a *Dendropanax morbifera* extract has the effect of improving the decrease of activity of visual cells in an animal model in which optic nerve cell damage was caused by the irradiation of an excessive light source and loss of optic nerve cells similar to dry macular degeneration was induced, the electroretinogram was measured.

Specifically, after allowing the animal model used in Experimental Example 5 to adapt to darkness for 16 hours or more the day before autopsy, they were subjected to inhalation anesthesia with isoflurane (Poran, JW Pharmaceutical), and their pupils were expanded using 0.5% tropicamide. An appropriate amount of 1% methylcellulose was added to the eyeballs while maintaining body temperature under general anesthesia, and the gold wire loops recording electrode was grounded to the cornea, and the reference electrode was placed on the cheek area, and the ground electrode was grounded to the tail, and then the electroretinogram was measured using Retiport (Roland Consult, Germany). After stimulation by irradiation with white LED light, wave a and wave b were obtained from the scotopic flash response and the wave a and wave b were measured by administering the test drug as described in Table 1 above to thereby measure the degree of activity of visual cells.

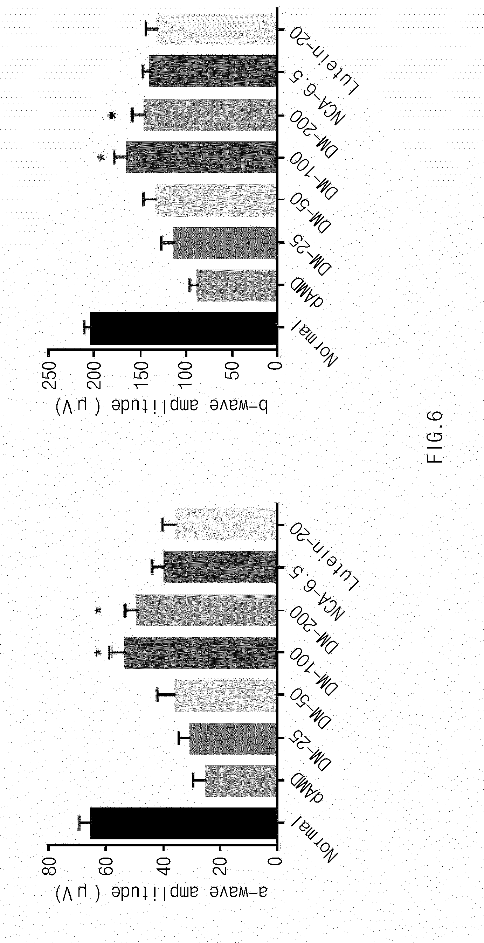

As a result, as shown in FIG. 6, it was confirmed that the administration of the *Dendropanax morbifera* extract improved the nerve conduction of the wave a and wave b and improved the decrease of activity of the visual cells, and the efficacy was superior to that of lutein. However, the effect of improving nerve conduction was slightly reduced in the experimental group administered with the *Dendropanax morbifera* extract at 200 mg/kg compared to the experimental group administered with the *Dendropanax morbifera* extract at 100 mg/kg, but the difference was not large. The group administered with neochlorogenic acid, which was another positive control group, also improved retinal nerve conduction, but it was not statistically significant.

From the above, it can be seen that the *Dendropanax morbifera* extract has the effect of improving the decrease of activity of visual cells.

### [Experimental Example 7]

### Effect of Dendropanax morbifera extract on improving damage or deformation of photoreceptor cells in animal model with macular degeneration

In order to confirm the effect of the *Dendropanax morbifera* extract on improving damage or deformation of photoreceptor cells, N-methyl-N-nitrosourea (MNU) toxic to photoreceptor cells was administered, and the changes in thickness of the outer nuclear layer (ONL) of the retina were measured in an animal model where loss of optic nerve cells similar to dry macular degeneration was induced.

Specifically, 6-week-old male BALB/c mice (Orient Bio Inc.) were acclimatized for one week, and N-methyl-N-nitrosourea (MNU) (Sigma, USA) was prepared to a concentration of 1% in a 0.05% acetic acid solution and was administered intraperitoneally to mice at a concentration of 1%, 60 mg/kg per individual, in which only the same amount of 0.05% acetic acid was administered intraperitoneally in the negative control. The test drug was orally administered once daily for 7 days from the same day after administration of *N*-methyl-*N-*nitrosourea. The concentrations of the drugs administered are shown in Table 2 below. Neochlorogenic acid, which is known to help prevent macular degeneration by activating aged retinal nerve cells, and lutein, which is widely known as a component of eye nutrients, were set as positive controls, and the *Dendropanax morbifera* extract according to Preparation Example above was administered at various concentrations. After the completion of drug administration, autopsies were performed to extract the eyeballs, which were fixed in 10% neutralized formalin for one day, and then embedded in paraffin to prepare slide sections. The slide sections were stained with hematoxylin & eosin (H&E) and observed under an optical microscope, and damage or deformation of photoreceptor cells was evaluated by measuring changes in the thickness of the outer nuclear layer of retinal tissue.

**[Table 2]**

| **Test Group** | **Group Name** | **Drug Administered** | **Dose Concentration (mg/kg/day)** | **No. of Mice** |
|---|---|---|---|---|
| Normal Control Group | Normal | - | - | 5 |
| Negative Control Group | MNU | - | - | 5 |
| Group Administered with *Dendropanax morbifera* Extract | DM-10 | *Dendropanax morbifera* extract | 10 | 5 |
| Group Administered with *Dendropanax morbifera* Extract | DM-25 | *Dendropanax morbifera* extract | 25 | 5 |
| Group Administered with *Dendropanax morbifera* Extract | DM-50 | *Dendropanax morbifera* extract | 50 | 5 |
| Group Administered with *Dendropanax morbifera* Extract | DM-100 | *Dendropanax morbifera* extract | 100 | 5 |
| Positive Control Group | NCA-6.5 | Neochlorogenic acid | 6.5 | 5 |
| Positive Control Group | NCA-13 | Neochlorogenic acid | 13 | 5 |
| Positive Control Group | Lutein | Lutein | 20 | 5 |

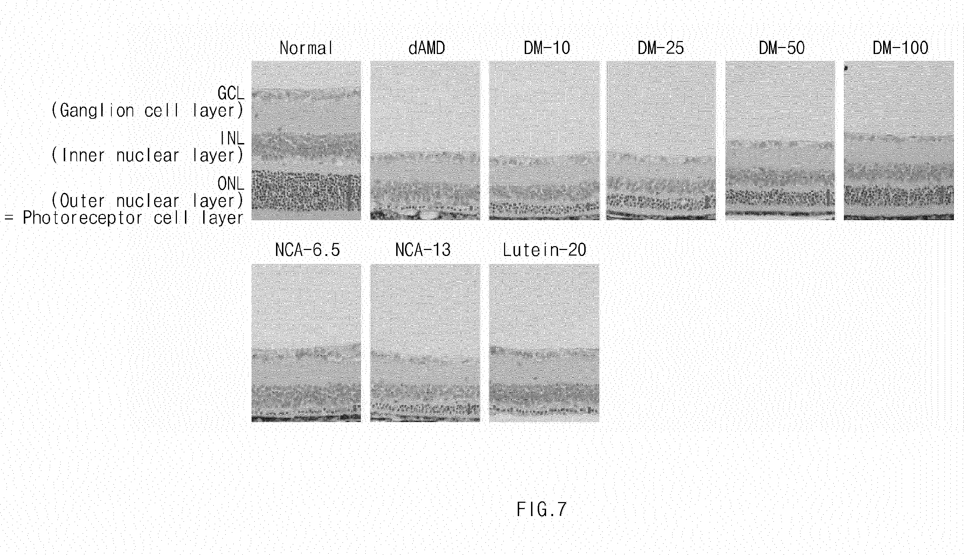

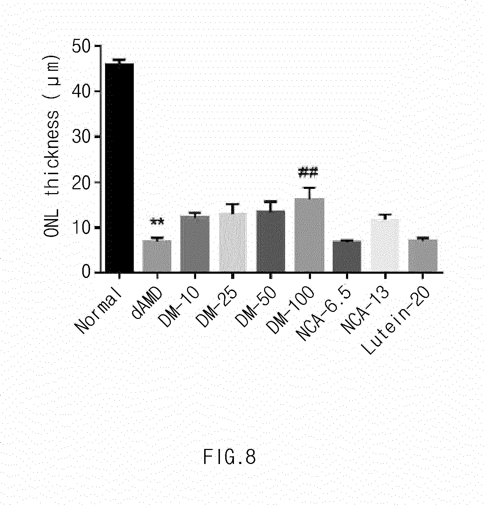

As a result, as shown in FIGS. 7 and 8, in the negative control group, the thickness was decreased while the cell nuclei of the outer nuclear layer (ONL) containing the nuclei of optic nerve cells was decreased, and when a *Dendropanax morbifera* extract was administered, it was shown that the thickness of the outer nuclear layer was increased in a concentration-dependent manner, and its effect was superior to that of lutein. The thickness of the outer nuclear layer was also increased in the group administered with neochlorogenic acid, which was another positive control group, but the result was not statistically significant. From the above, it can be seen that the *Dendropanax morbifera* extract has the effect of improving the damage or deformation of photoreceptor cells by increasing the thickness of the outer nuclear layer (ONL) of the retina.

### [Experimental Example 8]

### Effect of Dendropanax morbifera extract on improving damage of retinal pigment epithelial cell in animal model with macular degeneration

In order to determine whether a *Dendropanax morbifera* extract has the effect of improving damage of retinal pigment epithelial cells, the locations where the folding deformation of the retinal tissue occurred were counted and evaluated in an animal model, in which sodium iodate (NaIO₃) toxic to retinal pigment epithelial cells was administered to retinal pigment epithelial cells and thus morphological deformation was induced in the retinal tissue positioned on an upper part thereof.

Specifically, 6-week-old male SD rats (Orient Bio Inc.) were acclimatized for one week, and sodium iodate (NaIO₃) (Sigma, USA) to induce damage and degeneration of retinal pigment epithelial cells was prepared to a concentration of 3.5% in sterile physiological saline and was administered intraperitoneally to each rat in an amount of 60 mg/kg per individual, and only an equal amount of physiological saline was intraperitoneally administered in the negative control group. The test drug was orally administered once daily for 14 days from the same day after administration of sodium iodate. The concentrations of the drugs administered are shown in Table 3 below. Neochlorogenic acid, which is known to help prevent macular degeneration by activating aged retinal nerve cells, and lutein, which is widely known as a component of eye nutrients, were set as positive controls, and the *Dendropanax morbifera* extract according to Preparation Example above was administered at various concentrations. After the end of drug administration, autopsies were performed to extract the eyeballs, which were fixed in 10% neutralized formalin for one day, and then embedded in paraffin to prepare slide sections. The slide sections were stained with hematoxylin & eosin (H&E) and observed under an optical microscope, and damage or deformation of retinal pigment epithelial cells was evaluated by counting the locations where folding deformation of retinal tissue occurred.

**[Table 3]**

| **Test Group** | **Group Name** | **Drug Administered** | **Dose Concentration (mg/kg/day)** | **No. of Rats** |
|---|---|---|---|---|
| Normal Control Group | Normal | - | - | 5 |
| Negative Control Group | NaIO₃ | - | - | 5 |
| Group Administered with *Dendropanax morbifera* Extract | DM-25 | *Dendropanax morbifera* extract | 25 | 5 |
| Group Administered with *Dendropanax morbifera* Extract | DM-50 | *Dendropanax morbifera* extract | 50 | 5 |
| Group Administered with *Dendropanax morbifera* Extract | DM-100 | *Dendropanax morbifera* extract | 100 | 5 |
| Positive Control Group | NCA | Neochlorogenic acid | 6.5 | 5 |
| Positive Control Group | Lutein | Lutein | 50 | 5 |

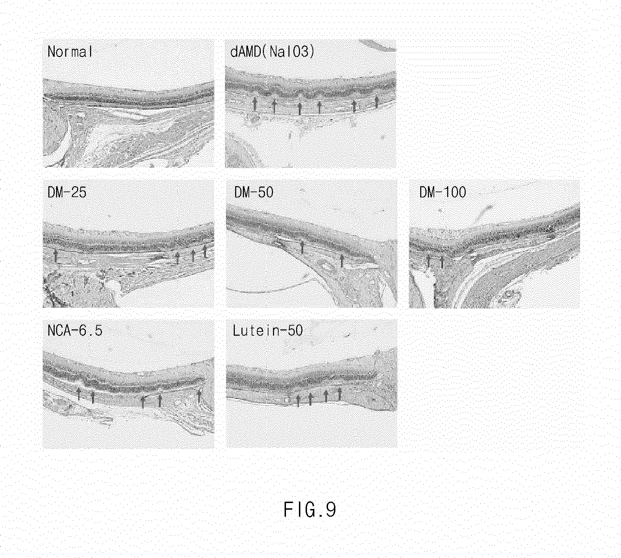

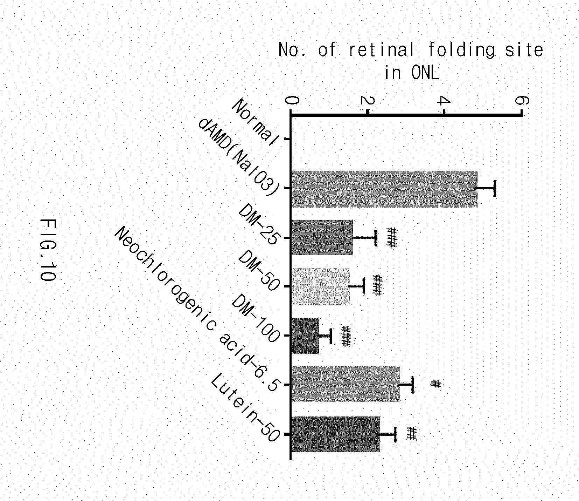

As a result, as shown in FIGS. 9 and 10, in the negative control group, retinal tissue was deformed due to damage to retinal pigment epithelial cells, and a number of folded positions (red arrows) were observed, whereas when the *Dendropanax morbifera* extract was administered, it was shown that the folded positions were inhibited due to tissue deformation that occurred in a concentration-dependent manner, and the efficacy was superior compared to lutein. From the above, it can be seen that the *Dendropanax morbifera* extract has the effect of inhibiting deformation of retinal tissue thereby improving damage or deformation of retinal pigment epithelial cells.

### [Experimental Example 9]

### Confirmation of action mechanism of effect of Dendropanax morbifera extract on improving macular degeneration

### [9-1] Confirmation of mechanism of action of Dendropanax morbifera extract on improving damage of optic nerve cells caused by light irradiation

In order to confirm the mechanism of action of the effect of improving optic nerve cell damage caused by light irradiation confirmed in Experimental Example 5, it was confirmed whether the damage and loss of optic nerve cells caused by excessive irradiation of a light source was due to apoptosis, and whether the effect of the *Dendropanax morbifera* extract on improving the damage or deformation of photoreceptor cells in an animal model with macular degeneration was due to anti-apoptotic effect.

Specifically, in the animal model of Experimental Example 5, the degree of apoptosis of optic nerve cells was analyzed using retinal tissue slides prepared for analysis of damage and loss of optic nerve cells. After completion of administration of the test drug, autopsies were performed to extract the eyeballs, which were fixed in 10% neutralized formalin for one day, and then embedded in paraffin to prepare slide sections. The tissue sections were hydrated by deparaffinization, washed with PBS, treated with a proteinase K solution at a concentration of 20 µg/mL at 37 °C for 15 minutes, and then washed again with PBS. After reacting in the TUNEL reaction mixture solution (*In situ* apoptosis detection kit, AP, Roche, Germany) at 37 °C for 1 hour, the resultant was observed under a microscope.

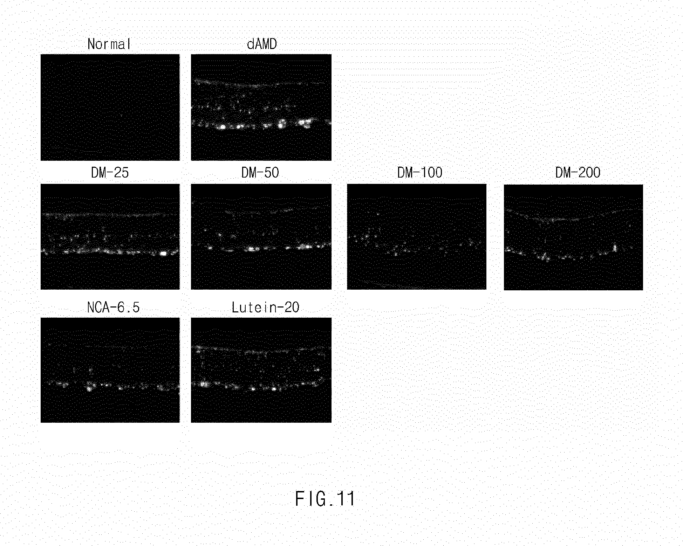

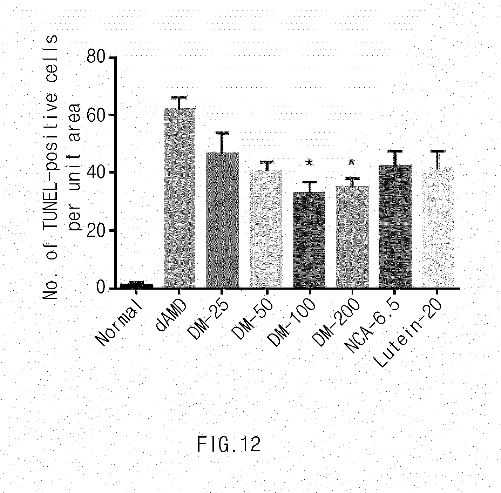

As a result, as shown in FIGS. 11 and 12, in the retina in which macular degeneration was induced through light irradiation, apoptosis was induced in all cells of the three layers (i.e., the Ganglion cell layer (GCL), the inner nuclear layer (INL), and the outer nuclear layer (ONL)), and in particular, a strong TUNEL positive reaction was confirmed in the optic nerve cell nuclei of the outer nuclear layer, confirming that apoptosis occurred mostly in the outer nuclear layer. However, it was confirmed that when treated with a *Dendropanax morbifera* extract, the apoptosis was inhibited by a decrease of the TUNEL-positive reaction in the cell nuclear layer of the optic nerve in a concentration-dependent manner, and the efficacy was superior to that of lutein.

From the above, it can be seen that the *Dendropanax morbifera* extract improves damage of optic nerve cells by inhibiting retinal apoptosis induced by light irradiation.

### [9-2] Confirmation of apoptosis inhibitory action mechanism of Dendropanax morbifera extract

In order to confirm the results confirmed in Experimental Example 9-1, the expression of proteins associated with apoptosis was confirmed in retinal tissue.

Specifically, the retinal tissue used in Experimental Example 9-1 was rapidly frozen in liquid nitrogen and then stored at -70 °C. After protein quantification of samples homogenized with homogenization buffer (pH 7.6), 30 µg each therefrom was subjected to electrophoresis in SDS-PAGE. Then, the proteins were transferred onto a PVDF membrane (Bio-Rad Laboratories, CA, USA) using transfer buffer (0.25 M tris, 1.92 M glycine, pH 8.3-8.4). After dissolving 5% nonfat milk in TBST (200 mM tris, 1.37 M NaCl, 0.05% tween 20) solution and blocking for 30 minutes, the primary antibodies were incubated at 4 °C, washed three times with TBST for 10 minutes each, and then reacted with HRP-bound secondary antibodies and washed again with TBST three times for 10 minutes each. Then, the resultant was reacted with an enhanced chemiliuminescence (ECL) solution, and the expression levels of Bax, Bcl-2, and caspase-3, which are apoptosis-related signaling materials, were confirmed with LAS-3000 (Fuji film, Japan).

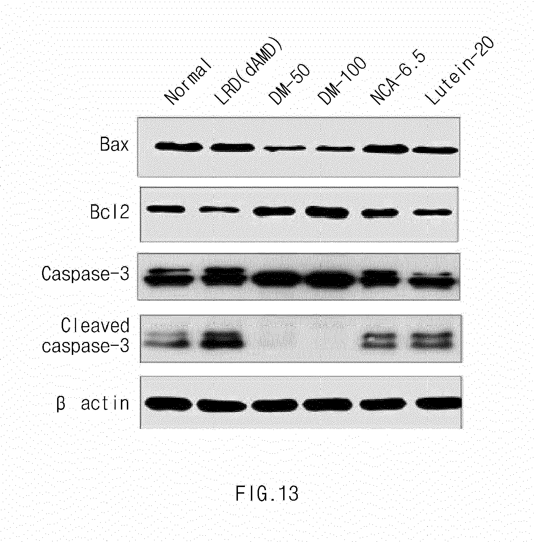

As a result, as shown in FIG. 13, in the case of Bax, which is a pro-apoptotic factor that induces apoptosis, the expression was significantly reduced by treating with a *Dendropanax morbifera* extract, whereas the expression of Bcl-2, which is an anti-apoptotic factor, was increased when treated with a *Dendropanax morbifera* extract. In addition, it was confirmed that when macular degeneration was induced, the expression of caspase-3 in a truncated form expressed during apoptosis was significantly increased, but the expression was decreased by the treatment with a *Dendropanax morbifera* extract.

From the above, it can be seen that the *Dendropanax morbifera* extract inhibits apoptotic factors while activating anti-apoptotic factors thereby improving damage of optic nerve cells.

### [9-3] Confirmation of mechanism of action of Dendropanax morbifera extract on improving damage of retinal pigment epithelial cells

When macular degeneration occurs, there occurs a problem with respect to damage and permeability of retinal pigment epithelial cells (RPE). In order to verify the inhibition of such damage and changes in permeability of retinal pigment epithelial cells, the changes in ZO-1, which is a cell-cell tight junction protein, were confirmed.

Specifically, in order to isolate the retinal pigment epithelial cell(RPE)-choroid-sclea complex containing the retinal pigment epithelium layer after removing the eyeballs during an autopsy, the lens, the anterior segments (anterior chambers), and the retinas were removed from the eyeballs while observing under a dissecting microscope and the posterior eyecups of the eyeballs were immersed in 4% paraformaldehyde (PFA) and fixed at 4 °C for one day. One day thereafter, the posterior eyecups were washed several times with PBS to remove the fixative and the eyecups were cut at four corners and spread flat on a slide glass. The resultants were treated with a blocking solution (10% normal goat serum) for 10 minutes, an anti-ZO-1 antibody (1:200, Cat# 61-7300, Invitrogen, Carlsbad, CA) for 1 hour, and then washed several times. Then, the resultant was treated with Alexa Fluor 488 goat-anti-rabbit (1:500; Cat# A-11008, Invitrogen) as a secondary antibody for 1 hour, and the flatted retinal pigment epithelial cell-choroid-sclera complex was examined under a fluorescence microscope (Olympus, Tokyo, Japan).

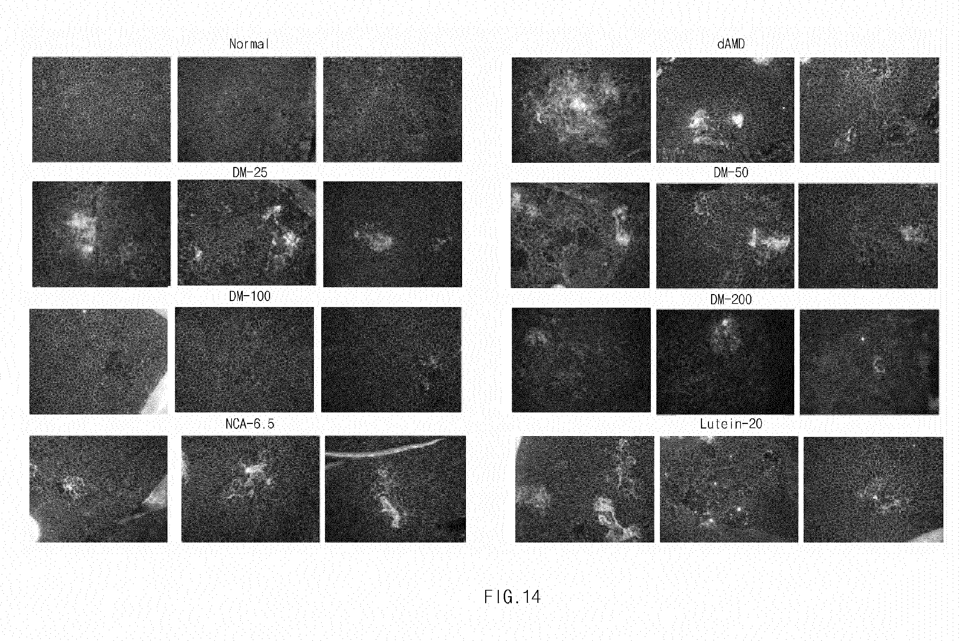

As a result, as shown in FIG. 14, it was confirmed that in the case of the normal group, no change was observed in the retinal pigment epithelial cell membrane, whereas in the group where sodium iodate (NaIO₃) was administered to cause morphological deformation of the retinal tissue group (dAMD), the size of retinal pigment epithelial cells was changed, and the cell membrane was collapsed. However, in the group treated with a *Dendropanax morbifera* extract, it was confirmed that changes in retinal pigment epithelial cells were inhibited in a concentration-dependent manner.

From the above, it can be seen that a *Dendropanax morbifera* extract improves the damage of retinal pigment epithelial cells by maintaining proteins constituting the tight junction of retinal pigment epithelial cells.

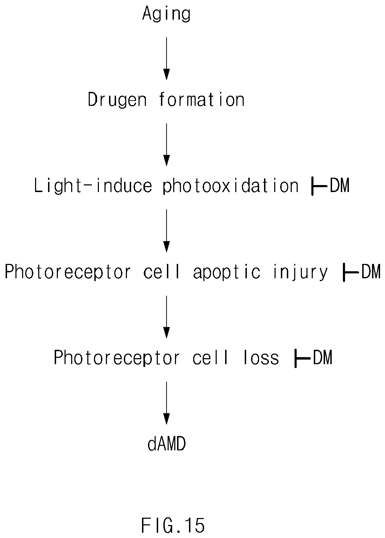

In summary, it can be seen that the *Dendropanax morbifera* extract not only inhibits damage to optic nerve cells through inhibition of photooxidation of drusen, which is a causative agent of macular degeneration, and inhibits apoptosis of optic nerve cells by inhibiting the expression of apoptosis signaling materials, but also maintains tight junction proteins in retinal pigment epithelial cells, thereby exhibiting the effect of improving macular degeneration (FIG. 15).

## Claims

1. A composition for preventing eye damage comprising a *Dendropanax morbifera* extract as an active ingredient.

2. The composition of claim 1, wherein the extract is extracted with water, C₁₋₄ alcohol, or a mixed solvent thereof.

3. The composition of claim 1, wherein the eye damage is caused by blue light.

4. The composition of claim 1, wherein the eye damage is caused by oxidative damage of eye, eye aging, eye fatigue, deterioration of an eye function, or an eye disease.

5. The composition of claim 4, wherein the eye disease is one or more types selected from the group consisting of retinal degeneration, macular degeneration, glaucoma, retinitis pigmentosa, Stargardt disease, choroideremia, gyrate-atrophy, retinal detachment, optic neuropathy, and dry eye syndrome.

6. The composition of claim 1, wherein the composition inhibits oxidation of *N*-retinylidene-*N*-retinyl-ethanolamine (A2E) by blue light, inhibits cell death by oxidized A2E, inhibits accumulation of drusen, increases the thickness of the outer nuclear layer (ONL) of the retina, improves the decrease in activity of visual cells, and inhibits deformation of retinal tissue.

7. A health functional food for preventing or improving eye damage comprising a *Dendropanax morbifera* extract as an active ingredient.

8. A pharmaceutical composition for preventing or treating an eye disease comprising a *Dendropanax morbifera* extract as an active ingredient.

9. The pharmaceutical composition of claim 8, wherein the eye disease is caused by blue light.

10. The pharmaceutical composition of claim 8, wherein the eye disease is one or more selected from the group consisting of retinal degeneration, macular degeneration, glaucoma, retinitis pigmentosa, Stargardt disease, choroideremia, and gyrate-atrophy, retinal detachment, optic neuropathy, and dry eye syndrome.
